(19) **Europäisches Patentamt**
**European Patent Office**
**Office européen des brevets**

(11) **EP 4 288 141 B1**

(12) # EUROPEAN PATENT SPECIFICATION

(45) Date of publication and mention
of the grant of the patent:
**21.08.2024   Bulletin 2024/34**

(21) Application number: **22777181.3**

(22) Date of filing: **26.04.2022**

(51) International Patent Classification (IPC):
*A61N 1/36* *(2006.01)*        *A61N 1/372* *(2006.01)*
*A61N 1/378* *(2006.01)*       *A61B 5/06* *(2006.01)*
*H02J 50/90* *(2016.01)*       *A61B 5/00* *(2006.01)*
*H02J 50/12* *(2016.01)*       *A61N 1/05* *(2006.01)*
*H02J 7/02* *(2016.01)*

(52) Cooperative Patent Classification (CPC):
**A61N 1/3605; A61B 5/0082; A61B 5/061;**
**A61N 1/37229; A61N 1/3787; H02J 50/12;**
**H02J 50/90;** A61N 1/0534; A61N 1/36053;
A61N 1/36128; H02J 7/02

(86) International application number:
**PCT/EP2022/060993**

(87) International publication number:
**WO 2023/208323 (02.11.2023 Gazette 2023/44)**

(54) **SYSTEM FOR OPTICALLY MEASURING A SUBCUTANEOUS IMPLANTATION DEPTH OF AN IMPLANT**

SYSTEM ZUR OPTISCHEN MESSUNG EINER SUBKUTANEN IMPLANTATIONSTIEFE EINES IMPLANTATS

SYSTÈME DE MESURE OPTIQUE D'UNE PROFONDEUR D'IMPLANTATION SOUS-CUTANÉE D'UN IMPLANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.12.2023   Bulletin 2023/50**

(73) Proprietor: **SYNERGIA MEDICAL**
**1435 Mont-Saint-Guibert (BE)**

(72) Inventors:
• **DOGUET, Pascal**
  **1435 Mont-Saint-Guibert (BE)**
• **GARNIER, Jérôme**
  **1435 Mont-Saint-Guibert (BE)**
• **RUEFF, Arnaud**
  **1435 Mont-Saint-Guibert (BE)**

(74) Representative: **Connor, Marco Tom et al**
**Pecher & Partners**
**Rue Louis de Geer, 6**
**1348 Louvain-la-Neuve (BE)**

(56) References cited:
**EP-A2- 1 882 484        WO-A1-2017/202455**
**US-A1- 2008 262 468     US-A1- 2014 203 823**
**US-B1- 9 672 393**

## Description

### TECHNICAL FIELD

The present invention concerns a system for determining an implantation depth of an active implantable medical device (AIMD) in a body of a patient. The implantation depth is measured with optical light beams emitted from the implanted AIMD and reaching a skin of the patient. Knowing the implantation depth is important to optimize the circuit of an external charger for charging a battery located in the AIMD, such as to approach, preferably reach resonance between an implanted secondary coil (= receiver) enclosed in the AIMD and an external primary coil (= emitter) in the external charger. Approaching, preferably reaching the resonance between the external charger and the AIMD is desired to reduce charging time of the battery and enhance the external charger autonomy.

### BACKGROUND OF THE INVENTION

Many types of active implantable medical devices (AIMD) exist and have various functions. Once implanted, they must cooperate with an external element to continue their function. In particular, for AIMD's consuming electrical energy and enclosing an implanted rechargeable battery, such as neurostimulators, the implanted battery must be replenished at regular intervals.

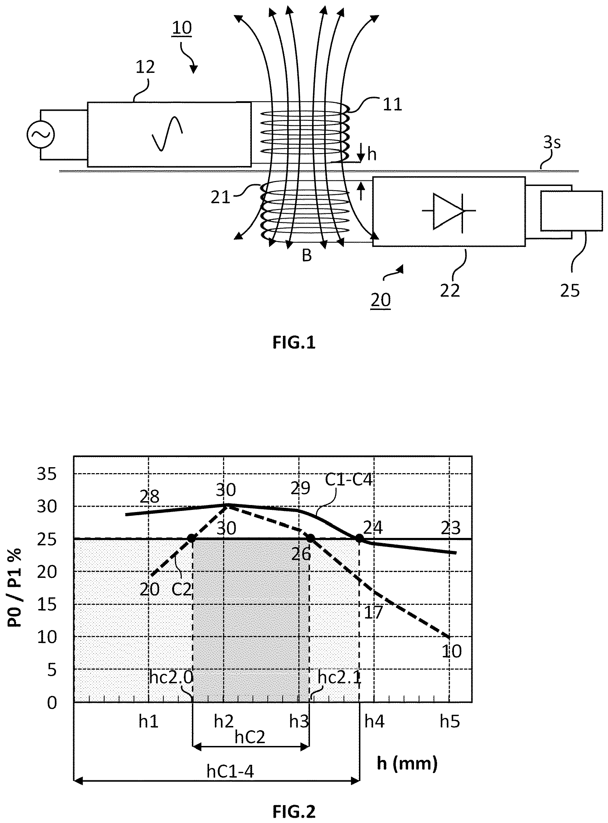

As shown in Figure 1, transcutaneous (wireless) charging of an implanted battery (25) lodged in an implanted AIMD (20) can be carried out across the skin (3s) of the patient with an external charger (10) by inducing an alternative current in a secondary coil (21) of the implanted AIMD by means of a magnetic field (B) generated by an alternative current circulating in a primary coil (11) of the external charger (10). In the following, unless otherwise specified, the term "current" is used to define an alternative current.

The power transfer efficiency (P0 / P1) defined as a ratio of the power (P0) received at the receiver (22) of the implanted AIMD, to the power (P1) consumed at the transmitter (12) of the external element (10) strongly depends on a number of parameters, including the alignment of the primary and secondary coils (11, 12), the tilting angle of deviation from parallelism between the two coils, and also the distance (h) separating the primary coil (11) from the secondary coil (22).

Positioning the external charger (10) to bring the primary coil (11) in perfect alignment with the secondary coil (21) can be achieved by means known in the art. For example, WO2017202455 describes a system for centring the primary coil (11) of an external charger (10) with respect to the secondary coil (21) of an implanted AIMD (20) by providing the implanted AIMD with a light source positioned such as to emit a light beam towards the skin of the patient, the light beam being coaxial to a secondary axis (Z2) centred on the secondary coil (21). The external charger comprises at least three photodetectors forming a polygon normal to and centred on the primary axis (Z1), which is itself centred on the primary coil (11). The primary and secondary coils (11, 21) are coaxial when an equal optical energy is received from the light beam by all the photodetectors of the external charger. Any difference in optical energy between two photodetectors is indicative of an offset between the primary and secondary axes (Z1, Z2).

In many applications, such as for vagus nerve stimulation (VNS) or for deep brain stimulation (DBS), wherein the AIMD is implanted, respectively, in the sub-clavicular area or between the skull and skin of the patient, the AIMD is generally substantially parallel to the skin and therefore so are the secondary coil (21) and the primary coil (11) of the AIMD (20) and external charger (10) (i.e., the tilting angle is substantially nil).

The power transfer efficiency (P01 P1) also depends on the distance (h) separating the primary coil (11) from the secondary coil (21). Each one of the transmitter circuit comprising the primary coil (11) and the receiver circuit comprising the secondary coil (21) includes one or more charging capacitors to yield capacitance values precisely tuned to enhance the resonance between the transmitter circuit and the receiver circuit. Resonance occurs when the natural frequency of a system is the same as the frequency of an oscillating force applied to the system. Its effect is to maximize the amplitude of the oscillating response of the system. The oscillating force is the electromagnetic force associated with the oscillating magnetic field (B) created by powering the primary coil (11) at a constant frequency. The natural frequency of the system is variable and depends on several parameters, including the distance (h) between the primary and secondary coils (11, 21). To enhance the resonance, the capacitance values of the external charger shall be adapted and tuned as a function of the actual distance (h). Since in a system composed of an external charger (10) laid on the skin of the patient over an implanted AIMD (20), the distance (h) is equal to, or univocally related to the value of the implantation depth of the AIMD, in the following, the terms "distance" (h) and "implantation depth" (h) are used interchangeably as synonyms.

Figure 2 illustrates the power transfer efficiency (P0 / P1) as a function of the implantation depth (h). The dashed line labelled "C2" characterizes the P0 / P1 vs h plot for a prior art external charger comprising a single charging capacitor (C2) of fixed capacitance. It can be seen that the power transfer efficiency (P01 P1) varies strongly with the implantation depth (h). Taking arbitrarily P0 / P1 = 25% (cf. horizontal straight line) as a minimum power transfer efficiency (P0 / P1) below which the power transfer is considered as not sufficiently efficient, it can be seen that the external charger of the prior art labelled "C2" can only be used in a limited C2-implantation depth-range (hC2) comprised between hc2.0 and hc2.1.

The implantation depth (h) which an AIMD will be implanted at is not known in advance and the optimization of the capacitance values of the external charger cannot be carried out during production of the AIMD in plant. In general, the external charger and AIMD are produced with capacitance values which are reasonably optimized for a limited range of implantation depths (h) which is statistically representative of actual implantation depths. The problem remains to enhance resonance in all cases, including the cases wherein the implantation depth falls out of the limited range (hC2) of implantation depths (h).

A skilled person faced with the foregoing problem must first find a way to assess the implantation depth (h) of the AIMD and, second, optimize the capacitance value of the external charger (10) to the thus assessed implantation depth to enhance resonance of the system.

WO2017202455 describes a system for centring an external element, typically an inductive charger, with an implanted AIMD by optical means. This system is very efficient for centring the external element with the implanted AIMD but is incapable of determining an implantation depth of the AIMD.

US20080262468 describes a method for remodelling a target tissue, such as a tympanic membrane, in an ear canal of an ear of a patient. In order to ensure that a tip of an injection tube is brought to a predefined distance from the target tissue, a range finder system is provided comprising two light sources coupled to the tip positioned at an angle such that their respective light beams cross each other at the predefined distance. An image capture device allows visualizing the two light spots projected on the target tissue. When the two spots merge to form a single spot, the injection tube is at the predefined distance and is blocked at that position. The range finder allows determining whether the tip of the injection tube is at or out of the predefined distance from the target tissue but does not allow measuring a distance separating the tip from the target tissue.

US20140203823 describes a method for estimating the implantation depth of an implanted AIMD, by first relating a communication efiiciency to an impedance matching of an antenna within the implanted AIMD. The impedance matching is then related to the depth of the implantable medical device within the tissue of the patient, a type of tissue in which the implantable medical device is implanted, or both. The impedance matching cannot, however, be reliably related to a depth within a tissue, because every patient sare different from one another.

The present invention proposes an original, reliable and simple solution for solving this dual problem of assessing the actual implantation depth (h) and optimizing the capacitance value of the external charger to the actual implantation depth. These and other advantages of the present invention are presented in the next sections.

## SUMMARY OF THE INVENTION

The present invention is defined in the appended independent claims. Preferred embodiments are defined in the dependent claims. In particular, the present invention concerns a system for optically measuring an implantation depth (d) of an active implantable medical device (AIMD) below a skin of a patient implanted with the AIMD, the system comprising the AIMD and an external optical depth measuring device.

The AIMD (20) comprises an encapsulation unit suitable for being subcutaneously implanted with a reference plane (R) of the encapsulation unit being substantially parallel to the skin of the patient when implanted in the patient and comprising a housing comprising walls defining an inner volume sealed from an outer environment by the walls and defining a first main surface configured for facing the skin of the patient when implanted in the patient. One or more light sources are provided, which are configured for emitting one or more light beams from the main surface towards the outer environment to form a light projection pattern at a level of the skin of the patient, characterized by an intensity profile comprising at least first and second maxima separated from one another by a maxima distance (ds).

The external optical depth measuring device (40) comprises a controller and two or more photodetectors. The controller is configured for activating the one or more light sources of the AIMD. The two or more photodetectors are configured for detecting the at least first and second maxima of the light projection pattern emitted by the one or more light sources.

The gist of the invention is that the one or more light sources are configured for emitting one or more light beams along at least two emission axes (X1, X2) defined as the axes extending from one or more emission points of the one or more light sources to the corresponding at least first and second maxima of the light projection pattern at the level of the skin of the patient. The at least two emission axes (X1, X2) are non-parallel to one another, and form at least first and second axis angles ($\alpha$1, $\alpha$2) relative to a normal axis (n1; n2) perpendicular to the reference plane (R), each comprised between 0° and 88°, preferably between 10° and 60°, more preferably between 20° and 45°. The external optical depth measuring device comprises an intelligence configured for,

- determining the respective positions of the first and second maxima,

- measuring the maxima distance (ds) between the first and second maxima at the level of the skin of the patient, and

- determining from the maxima distance (ds) the implantation depth (h) of the AIMD below the skin of the patient.

The implantation depth (h) can be determined with any one of the following expressions (1) or (1a),

$$h = \frac{ds}{\tan \alpha 1 + \tan \alpha 2} - h0, \text{ or} \qquad (1)$$

$$h = \frac{ds - d0}{\tan \alpha 1 + \tan \alpha 2} \qquad (1a)$$

wherein

- h0 is a distance measured along a central normal axis (n0) perpendicular to the reference plane (R) between the intersection point of the first and second emission axes (X1, X2) and the main surface (20m), and
- d0 is a distance separating the first from the second light sources (51, 52).

In a preferred embodiment, the first and second axis angles ($\alpha 1$, $\alpha 2$) are equal (i.e., ($\alpha 1$ = $a2$). By introducing $\alpha 1$ = $a2$ into Equations (1) or (1a), the implantation depth (h) can be determined with any one of the following expressions (3) or (3a),

$$h = \frac{ds}{2 \tan \alpha 1} - h0 \qquad (3)$$

$$h = \frac{ds - d0}{2 \tan \alpha 1} \qquad (3a)$$

In an alternative embodiment, the first axis angle ($\alpha 1$) is equal to zero (i.e., $\alpha 1$ = 0) and is different from the second axis angle ($\alpha 2$). Introducing $\alpha 1$ = 0 into Equations (1) or (1a), the implantation depth (h) can be determined with any one of the following expressions (4) or (4a),

$$h = \frac{ds}{\tan \alpha 2} - h0 \qquad (4)$$

$$h = \frac{ds - d0}{\tan \alpha 2} \qquad (4)$$

The one or more light sources can be formed either by,

- at least first and second light sources separated from one another by a distance (d0) and each configured for emitting a light beam along the corresponding at least first and second emission axes (X1, X2), or

- a single light source configured for emitting a light beam characterized by the at least first and second maxima.

In a preferred embodiment, the system comprises an external charger comprising or connectable to a source of power for feeding alternative current to a primary coil made of one or more wires coiled about a primary axis (Z1) and forming an external emitter of magnetic waves. In this embodiment, the AIMD comprises a secondary coil made of one or more wires coiled about a secondary axis (Z2) normal to the reference plane (R) and forming an internal receiver. With this configuration, when the electric charger is located adjacent to the AIMD with the primary axis (Z1) positioned coaxially to the secondary axis (Z2) and when the primary coil is fed with alternative current, a magnetic link is formed and an alternative current induced in the secondary coil is optimized, which serves to power the AIMD or to recharge a rechargeable battery enclosed in the inner volume (Vi).The external charger can comprise,

- an electrical circuit configured for varying the properties of the alternative current fed to the primary coil,
- a processor configured for retrieving the implantation depth (h) of the AIMD measured by the external optical depth measuring device and for controlling the alternative current fed to the primary coil to optimize a power transfer efficiency between the primary coil of the external charger and the secondary coil of the AIMD as a function of the implantation depth (h).

In a preferred embodiment, the external optical depth measuring device is included in the external charger and the intelligence is included in the processor.

In particular, the electrical circuit can comprise at least first and second charging capacitors (C1, C2) of given capacitances, which can be individually connected or disconnected to the electrical circuit. The processor can be configured for optimizing the power transfer efficiency between the primary coil of the external charger and the secondary coil of the AIMD as a function of the implantation depth (h) by selecting which of the first and / or second capacitance (C1, C2) to connect to the electrical circuit.

In a preferred embodiment, the electrical circuit comprises N charging capacitors (C1-CN) of same or different charging capacitors, which can be connected or disconnected individually or in groups of charging capacitors to the electrical circuit. The processor can be configured for determining which combination of one or several of the N charging capacitors (C1-CN) to connect to the electrical circuit, such as to optimize the power transfer efficiency as a function of the implantation depth (h). the number N of capacitors can be comprised between 3 and 8, preferably between 4 and 6.

The present invention also concerns a method for determining an implantation depth (h) separating an external optical measuring device from an active implantable medical device (= AIMD) implanted in a patient, comprising the following steps,

- providing an AIMD as defined supra, whose main surface is separated from an outer environment by a distance to be determined, by a skin substantially parallel to the reference plane (R),

- applying an external depth measuring device as defined supra onto the skin, vis-à-vis the AIMD,

- activating with the controller the one or more light sources of the AIMD,

- measuring the maxima distance (ds) and determining therefrom the value of the distance.

For optimizing a power transfer efficiency between a primary coil of an external charger and a secondary coil of the AIMD as a function of the implantation depth (h), the method mat further comprise,

- providing an external charger as defined supra,

- retrieving the thus determined distance, and

- determining an optimal alternative current to be fed to the primary coil as a function of the distance, to optimize the power transfer efficiency (P0 / P1) between the primary coil of the external charger and the secondary coil of the AIMD,

- applying the external charger onto the skin vis-à-vis the AIMD and feeding the optimal alternative current to the primary coil.

Controlling the alternative current fed to the primary coil can comprise selecting one or more charging capacitors (C1-CN) to connect to the electrical circuit, such as to optimize the power transfer efficiency between the primary coil of the external charger and the secondary coil of the AIMD as a function of the distance thus determined.

## BRIEF DESCRIPTION OF THE FIGURES

For a fuller understanding of the nature of the present invention, reference is made to the following detailed description taken in conjunction with the accompanying drawings in which:

Figure 1: shows the principle of inductive charging of a battery lodged in an implanted AIMD by means of an external charger.

**Figure 2:** compares the power transfer efficiency (P01 P1) between a system of the prior art with a single charging capacitor (C2) of constant capacitance value (= dashed line) with a system of the present invention comprising four charging capacitors (C1-C4) in parallel, allowing the capacitance of the electrical circuit of the external charger to be optimized as a function of the implantation depth (h).

**Figure 3(a):** illustrates the geometric principle of implantation depth measurement with the system of the present invention, with two light maxima for any value of $\alpha 1$ and $\alpha 2$.

**Figure 3(b):** plots the implantation depth (h) as a function of the measured maxima distance (ds) according to the Equation (1a).

**Figure 3(c):** plots the (ds / h) ratio of the maxima distance (ds) to the implantation depth (h) as a function of the value of the second axis angle ($\alpha 2$) for $\alpha 1$ = 30°.

**Figure 4:** illustrates the geometric principle of implantation depth measurement with the system of the present invention, with two light maxima for the special case of $\alpha 1 = \alpha 2$.

**Figure 5:** illustrates the geometric principle of implantation depth measurement with the system of the present invention, with two light maxima for the special case of $\alpha 1$ = 0°.

**Figure 6:** illustrates the geometric principle of implantation depth measurement with the system of the present invention with an annular light beam.

**Figure 7(a):** illustrates an external optical depth measuring device according to the present invention laid against the skin of a patient facing an implanted AIMD.

**Figure 7(b):** illustrates an external optical depth measuring device according to the present invention integrated in an external charging unit laid against the skin of a patient facing an implanted AIMD.

**Figure 8(a):** shows a representation of a simplified electrical circuit of an external charger according to the prior art.

**Figure 8(b):** shows a representation of a simplified electrical circuit of an external charger according to the present invention.

**Figure 9** shows a flowchart illustrating the optimization of the external charger after having determined the implantation depth (h) of the AIMD.

## DETAILED DESCRIPTION OF THE INVENTION

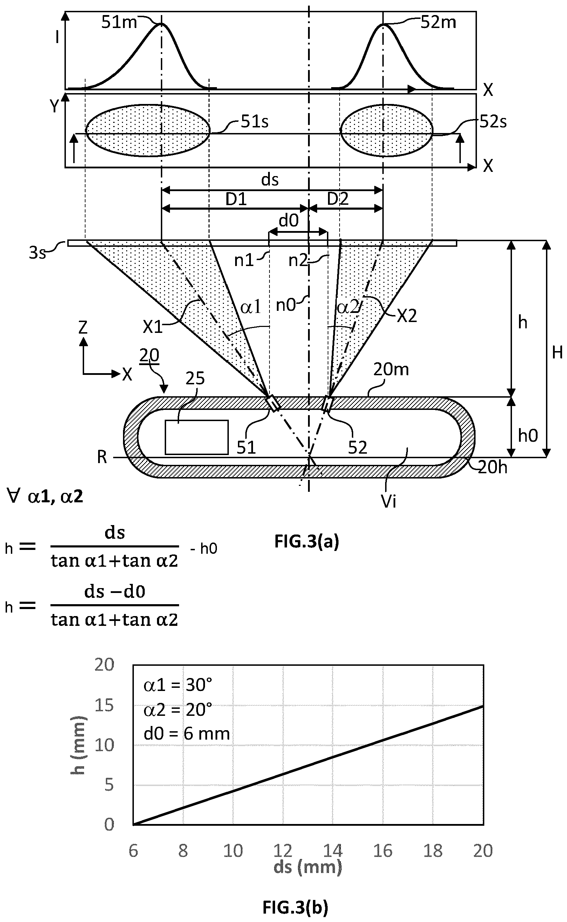

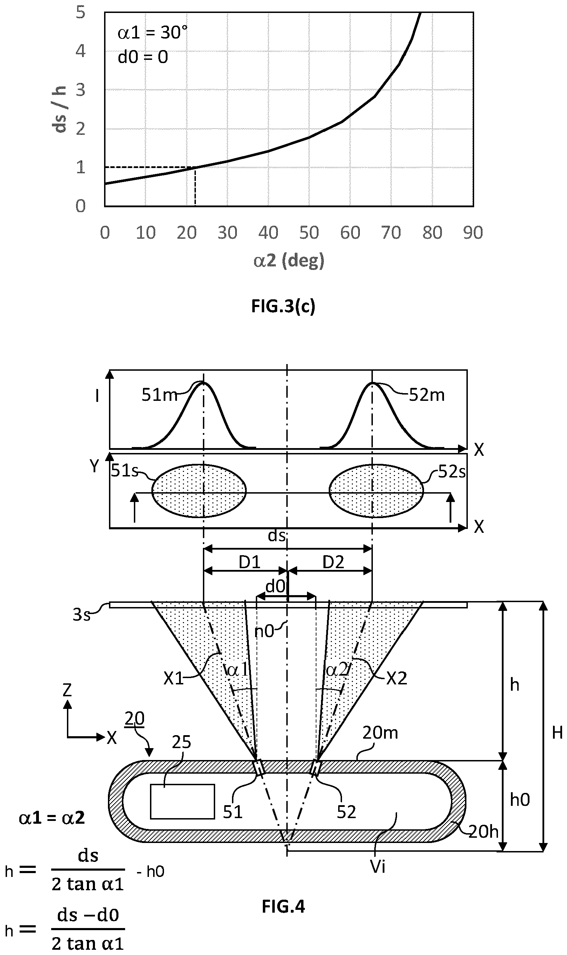

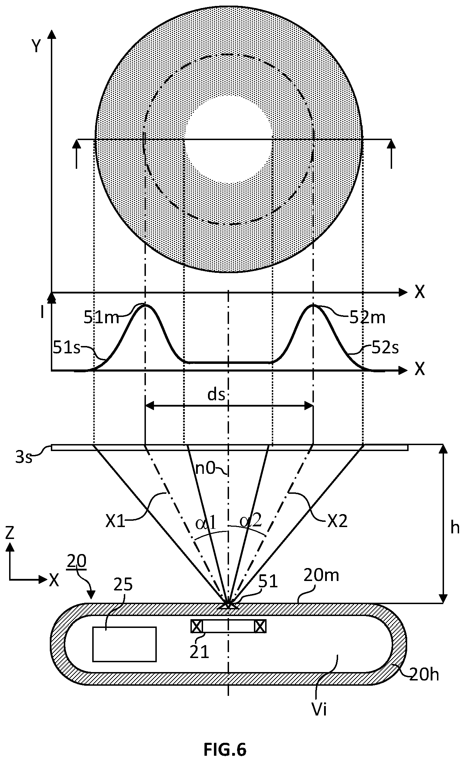

As illustrated in Figures 3 to 7, the system of the present invention is suitable for optically measuring an implantation depth (h) of an active implantable medical device (AIMD) (20) below a skin (3s) of a patient implanted with the AIMD. The system of the present invention comprises an AIMD and an external optical depth measuring device (40).
The AIMD (20) comprises an encapsulation unit (20e) and one or more light sources (51, 52). The encapsulation unit (20e) is suitable for being subcutaneously implanted with a reference plane (R) of the encapsulation unit being substantially parallel to the skin (3s) of the patient when implanted in the patient. It comprises a housing (20h) comprising walls defining an inner volume (Vi) sealed from an outer environment by the walls and defining a first main surface (20m) configured for facing the skin of the patient when implanted in the patient.
The one or more light sources (51, 52) are configured for emitting one or more light beams from the main surface (20m) towards the outer environment to form a light projection pattern (51s, 52s) at a level of the skin (3s) of the patient. The light projection pattern is characterized by an intensity profile comprising at least first and second maxima (51m, 52m) separated from one another by a maxima distance (ds). The AIMD can comprise first and second light sources (51, 52) as illustrated in Figures 3 to 5, which are separated from one another by a distance (d0) and each configured for emitting a light beam along the corresponding at least first and second emission axes (X1, X2). Alternatively, as shown in Figure 6, the AIMD can comprise a single light source (51) configured for emitting a light beam characterized by the at least first and second maxima (51m, 52m). The single light source can emit two or more discrete light beams or a single light beam forming a ring as shown in Figure 6
The external optical depth measuring device (40) comprises a controller configured for activating the one or more light sources of the AIMD, and two or more photodetectors (44) or an array of two or more photodetectors configured for detecting the at least first and second maxima (51m, 52m) of the light projection pattern emitted by the one or more light sources.

The one or more light sources are configured for emitting one or more light beams along at least two emission axes (X1, X2) defined as the axes extending from one or more emission points (51, 52) of the one or more light sources to the corresponding at least first and second maxima (51m, 52m) of the light projection pattern (51s, 52s) at the level of the skin (3s) of the patient. The at least two emission axes (X1, X2) are non-parallel to one another and form at least first and second axis angles ($\alpha$1, $\alpha$2) relative to a normal to the reference plane (R), each comprised independently from one another between 0° and 88°, preferably between 10° and 60°, more preferably between 20° and 45°.

The external optical depth measuring device comprises an intelligence (48) configured for,

- determining the respective positions of the first and second maxima (51m, 52m),
- measuring the maxima distance (ds) between the first and second maxima (51m, 52m) at the level of the skin (3s) of the patient, and
- determining from the maxima distance (ds) the implantation depth (h) of the AIMD below the skin (3s) of the patient.

Once the implantation depth (h) has been assessed, an external charger (10) can be tuned to enhance resonance of the system between the primary and secondary coils (11, 21) of the external charger and AIMD, respectively. To this effect, the external charger (10) can comprise an electrical circuit (16) configured for varying the properties of the electric current fed to the primary coil (11). A processor (19) is configured for retrieving the implantation depth (h) of the AIMD (20) measured by the external optical depth measuring device (40) and for controlling the current fed to the primary coil (11) to optimize the power transfer efficiency between the primary coil (11) of the external electric charger (10) and the secondary coil (21) of the AIMD (20) as a function of the implantation depth (h). The current fed to the primary coil (11) can be controlled by varying the capacitance of the one or more charging capacitors (C1C4) of the transmitter circuit and / or the power supplied to the transmitter circuit.

**AIMD (20)**

The active implantable medical device (20) (= AIMD) of the present invention comprises an encapsulation unit (20e) suitable for being subcutaneously implanted with a reference plane (R) of the encapsulation unit being substantially parallel to the skin (3s) of the patient when implanted in the patient. As illustrated in Figures 3(a),4 to 6, and 7(a), the encapsulation unit comprises a housing (20h) comprising walls defining an inner volume (Vi) sealed from an outer environment by the walls and defining a first main surface (20m) configured for facing the skin of the patient when implanted in the patient.

According to the present invention, the encapsulation unit comprises one or more light sources (51, 52) configured for emitting one or more light beams from the main surface (20m) towards the outer environment to form a light projection pattern (51s, 52s) at a level of the skin (3s) of the patient, which is substantially parallel to the main surface (20m). As shown in Figures 3 to 6, the light projection pattern is characterized by an intensity profile comprising at least first and second maxima (51m, 52m) separated from one another by a maxima distance (ds). The one or more light sources are configured for emitting one or more light beams along at least two emission axes (X1, X2) defined as the axes extending from one or more emission points of the one or more light sources (51, 52) to the corresponding at least first and second maxima (51 m, 52m) of the light projection pattern (51s, 52s) at the level of the skin (3s) of the patient. The at least two emission axes (X1, X2) are non-parallel to one another, and form at least first and second axis angles ($\alpha$1, $\alpha$2) relative to a normal to the reference plane (R), each comprised between 0° and 88°, preferably between 10° and 60°, more preferably between 20° and 45°.a

As shown in Figure 6, a single light source can be used to emit two or more beams along the at least two emission axes (X1, X2). In Figure 6, the single light source (51) is configured for emitting an infinite number of beams forming a continuous ring of light (cf. shaded areas of the doughnut shaped ring of light on top of Figure 6. Figure 6 shows a continuous ring which is circular but the light projection pattern can have other geometries such as a triangle or a rectangle, preferably a square, or any polygonal geometry. Alternatively, the single light source (51) can be configured to emit a finite number of at least two beams extending along at least corresponding first and second emission axes (X1, X2).

As shown in Figures 3 to 5 and 7, the encapsulation unit can comprise at least two distinct light sources (51, 52). The discussion of Figures 3 to 5 is based on two distinct light sources, but it is clear that the encapsulation unit can comprise more than two distinct light sources.

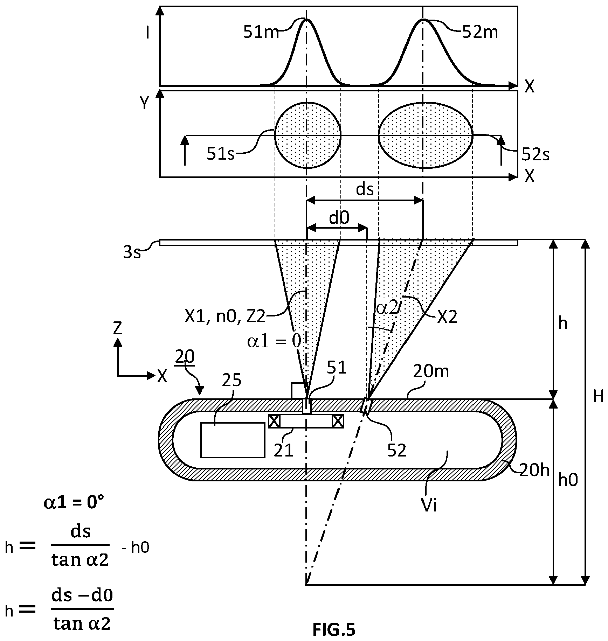

In Figure 3(a), the first and second axis angles ($\alpha$1, $\alpha$2) between the central normal axis (n0) perpendicular to the reference plane (R) and each of the first and second emission axes (X1, X2) can take any value comprised between 0° and 88°. Figure 4 shows an embodiment wherein the first axis angle ($\alpha$1) is equal to the second axis angle ($\alpha$2), i.e. $\alpha$1 = $\alpha$2. Figure 5 shows an embodiment wherein the first axis angle ($\alpha$1) is nil, i.e. $\alpha$1 = 0. The special embodiments of Figures 4 and 5 simplify the equation for determining the implantation depth (h) as a function of the maxima distance (ds) as is discussed in detail in continuation.

The intensity of the one or more light sources (51, 52) must be sufficient to give clear first and second light projection patterns with clearly distinguishable and identifiable maxima, so as to yield an accurate value of the maxima distance (ds). Since the tissues separating the AIMD from the skin of the patient can be inhomogeneous, light is not only absorbed but also scattered as it travels through the tissues. The duration of the light beams can, however, be very short, since all that is required is to identify the positions of the first and second maxima (51m; 52m) by the photodetectors (44). As the light beam must have a sufficient intensity, saving battery power can be achieved by shortening the duration of the light beam.

The AIMD can be a neurostimulator for stimulating electrically or optically a tissue such as a nerve. For example, the AIMD can be a neurostimulator for vagus nerve stimulation (VNS) or deep brain stimulation (DBS). The encapsulation (20e) of such AIMD's is generally implanted at a distance from the tissue to be stimulated and comprises a tissue coupling unit separate from the encapsulation unit (20e) and configured for coupling an electrode unit or an optrode unit to the tissue. Energy pulses are generated from an implanted pulse generator (IPG) lodged in the inner volume (Vi) of the encapsulation unit (20e). The energy pulses are transferred to the tissue coupling unit via electric wires or optical fibres, such as described in WO2016131492.

As shown in Figures 5, 6, 7(a) and 7(b), the AIMD (20) comprises a secondary coil (21) made of one or more wires coiled about a secondary axis (Z2) normal to the reference plane (R) and forming part of an implanted receiver (22). It is conductively coupled to an implanted battery (25) and configured for charging the implanted battery when exposed to an alternative magnetic field (B) inducing an electric current through the secondary coil (21). The alternative magnetic field can be generated by an external charger (10) comprising a primary coil (11) made of one or more wires coiled about a primary axis (Z1) and forming an external emitter (12) of magnetic waves, as discussed in continuation.

## EXTERNAL OPTICAL DEPTH MEASURING DEVICE (40)

The external optical depth measuring device (40) comprises a controller enclosed in a device housing (40h) and configured for activating the one or more light sources of the AIMD. In use, the external optical depth measuring device is laid onto the skin of the patient over the implantation location of the AIMD. In that position, the controller can communicate with the implanted AIMD and control the activation of the one or more light sources.

The external optical depth measuring device (40) comprises two or more photodetectors (44) or an array of two or more photodetectors configured for detecting the at least first and second maxima (51m, 52m) of the light projection pattern on the skin, emitted by the one or more light sources. The two or more photodetectors are preferably distributed on a single plane. In use, i.e., when the external optical depth measuring device (40) lies on the skin of the patient, over the AIMD, the single plane is preferably substantially parallel to the reference plane (R) of the AIMD. In case it is suspected or known that the AIMD is implanted such that the reference plane (R) thereof is not parallel to the skin, the tilting angle of the reference plane should be determined to correct the errors in implantation depth (h) determination due to the lack of parallelism of the single plane of the external optical depth measuring device (40) and the reference plane (R) of the AIMD (20). The external optical depth measuring device needs not be perfectly aligned with the AIMD, as long as the photodetectors (44) can detect at least first and second light (51m, 52m) maxima.

The external optical depth measuring device (40) also comprises an intelligence (48) configured for,

- determining the respective positions of the first and second maxima (51m, 52m),

- measuring the maxima distance (ds) between the first and second maxima (51m, 52m) at the level of the skin (3s) of the patient, and

- determining from the maxima distance (ds) the implantation depth (h) of the AIMD below the skin (3s) of the patient, in ways as described in continuation.

The thus determined maxima distance (ds) is important to tune the external charger (10) for inductively charging the implanted battery of the implanted AIMD as is explained in continuation.

## DETERMINATION OF THE IMPLANTATION DEPTH (h)

The implantation depth (h) can be determined trigonometrically from the maxima distance (ds) separating two intensity maxima (51m, 52m) of the light projection pattern (51s, 52s) at the level of the skin (3s) of the light beams extending along the first and second emission axes (X1, X2). Figure 3(a) illustrates a general case, wherein two separate light sources emit light beams along the first and second emission axes (X1, X2) forming axis angles ($\alpha$1, $\alpha$2), which

can take any value comprised between 0 and 88°.

The maxima distance (ds) is equal to the sum of D1 and D2 (i.e., ds = D1 + D2). From Figure 3(a) it can easily be assessed that D2 = D1 (tan $\alpha$2 / tan $\alpha$1). It follows that D1 = ds / (1+(tan $\alpha$2 / tan $\alpha$1)). Introducing this expression for D1 into the Equation, H = h + h0 = D1 / tan $\alpha$1 and rearranging yields Equation (1) which defines the implantation depth (h) as a function of the measured maxima distance (ds),

$$h = \frac{ds}{\tan \alpha 1 + \tan \alpha 2} - h0 \qquad (1)$$

The parameters, $\alpha$1, $\alpha$2, and h0 are known from the AIMD's manufacturer. If the value of h0 is not readily available, it can easily be determined as a function of the distance (d0) separating the first and second emission points (51, 52) in a similar way as Eq.(1) yielding,

$$h0 = \frac{d0}{\tan \alpha 1 + \tan \alpha 2} \qquad (2)$$

Introducing Eq. (2) into Eq.(1) yields Eq.(1a) expressing h as a function of ds, d0, and $\alpha$1, *a*2,

$$h = \frac{ds - d0}{\tan \alpha 1 + \tan \alpha 2} \qquad (1a)$$

Equations (1) and (1a) are valid for all values of $\alpha$1 and $\alpha$2 comprised between 0° and 88° independently of one another. The height (h0) is defined as the distance measured along a central normal axis (n0) perpendicular to the reference plane (R) between the intersection point of the first and second emission axes (X1, X2) and the main surface (20m). In case a single light source (51) emits beams along at least the first and second emission axes (X1, X2), the value of both h0 and d0 is zero (i.e., d0 = h0 = 0), as shown in Figure 6. Equations (1) and (1a) then simplify to Equation (1b),

$$h = \frac{ds}{\tan \alpha 1 + \tan \alpha 2} \qquad (1b)$$

Equations (1) and (1a) define a linear relationship between the measured maxima distance (ds) and the implantation depth (h). Figure 3(b) illustrates an Example of curve relating the implantation depth (h) to the maxima distance (ds), with $\alpha$1 = 30°, $\alpha$2 = 20°, and d0 = 6 mm.

The axis angles ($\alpha$1, $\alpha$2) can be comprised between 2 and 88°, independently of one from the other. Figure 3(c) plots the ratio (ds / h = tan $\alpha$1 + tan $\alpha$2) as a function of the axis angle $\alpha$2 (with $\alpha$2 = 30° and d0 = 0). It can be seen from Figure 3(c), that large axis angles ($\alpha$1, $\alpha$2) have the effect of increasing the ratio ds / h, which enhances the sensitivity of the measurement of ds for the determination of h, which is good for the accuracy of the determination of the implantation depth (h). The dashed line identifies ds = h. It is preferred that the axis angles ($\alpha$1, $\alpha$2) be selected such that the ratio ds / h $\geq$ 1.

The light beams must travel through tissues before reaching the skin and forming the light projection patterns (51s, 52s). As the path length through tissues the light beams must cross increases, the light projection patterns (51s, 52s). become weaker and more blurred, because of light absorption and scattering through non-homogenous tissues. Large axis angles increase the length of the path the beam lights must travel through the tissues before reaching the skin of the patient which therefore reduces the sharpness of the light projection patterns (51s, 52s). To maintain an agreeable sharpness of the maxima (51 m, 52m), it is therefore preferred that each axis angle ($\alpha$1, $\alpha$2) be not more than 60°, preferably not more than 45°. The shortest path from the main surface (20m) to the skin is defined by an axis angle, $\alpha$i = 0°, as shown in Figure 5.

**Special Case #1: $\alpha$1 = $\alpha$2**

As illustrated in Figures 4 and 6, in case the at least first and second emission axes (X1, X2) form a same axis angle (i.e., $\alpha$1 = $\alpha$2) with a normal axis (ni), Equations (1) and (1a) simplify to Equations (3) and (3a) as follows,

$$h = \frac{ds}{2 \tan \alpha 1} - h0 \qquad (3)$$

$$h = \frac{ds - d0}{2 \tan \alpha 1} \qquad (3a)$$

This configuration (i.e., $\alpha 1 = \alpha 2$) is particularly interesting in case more than two light sources distributed at equal distances from a central point and forming a polygon projecting homothetically the light projection pattern (51s, 52s) onto the skin. It is also interesting for an infinite number of beams extending along a corresponding infinite number of emission axes (X1, X2) homothetically forming a circular light projection pattern (51s, 52s) as shown in Figure 6. In these two embodiments, having first and second axis angles having a same value (i.e., $\alpha 1 = \alpha 2$) renders the implantation depth measurement independent of the angle of rotation over the central normal axis (n0) of the external optical depth measuring device (40) relative to the AIMD.

In the embodiment illustrated in Figure 6 a single light source projects a closed ring onto the skin (3s). With h0 = 0 and d0 = 0 (e.g., single light source), Equations (3) and (3a) simplify to Equation (3b),

$$h = \frac{ds}{2 \tan \alpha 1} \qquad (3b)$$

**Special Case #2: $\alpha 1 = 0°$**

As shown in Figure 5, the first axis angle can be nil (i.e., $\alpha 1 = 0°$). In other words, the first emission axis (X1) is perpendicular to the reference plane (R). It is mandatory that the second axis angle, $\alpha 2 \neq 0°$. Since tan 0 = 0, Equations (1) and (1a) simplify to Equations (4) and (4a) as follows,

$$h = \frac{ds}{\tan \alpha 2} - h0 \qquad (4)$$

$$h = \frac{ds - d0}{\tan \alpha 2} \qquad (4a)$$

This configuration (i.e., $\alpha 1 = 0°$) is preferred for various reasons. First, the first light source (51) having an axis angle, $\alpha 1 = 0°$, yields the shortest path to the skin of the patient and can therefore advantageously be used also for communicating with an external controller. Second, if the external charger (10) is as described in WO2017202455 provided with an optical centring system discussed more in detail in continuation, requiring the AIMD to emit a light along an emission axis forming an axis angle $\alpha 1 = 0°$, the same first light source (51) can be used for first determining the implantation depth (h) of the AIMD, and then aligning the external charger such that the primary coil (11) and secondary coil (21) be centred coaxially (i.e., Z1 = Z2).

In case a single light source is used, h0 = d0 = 0 and Equations (4) and (4a) simplify to Equation (4b),

$$h = \frac{ds}{\tan \alpha 2} \qquad (4b)$$

**EXTERNAL CHARGER (10)**

The determination of the implantation depth (h) of the AIMD is advantageous for tuning the external charger (10) so as to optimize resonance between the primary and secondary coils (11, 21) of the external charger and the AIMD, respectively. The external electric charger (10) is connectable to a source of electric current for feeding an alternative electric current to a primary coil (11) made of one or more wires coiled about a primary axis (Z1) and forming an external emitter (12) of magnetic waves.

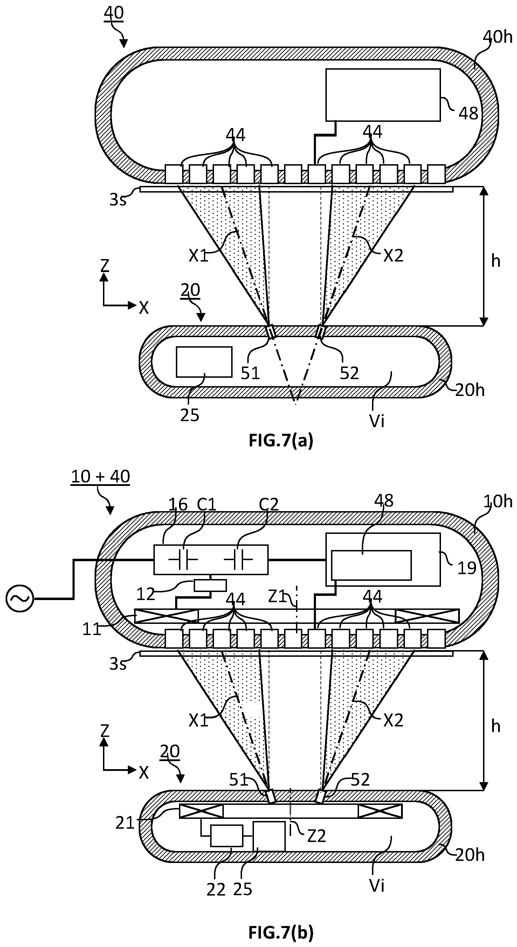

As described supra and illustrated in Figures 5, 6, 7(a), and 7(b), the AIMD (20) comprises a secondary coil (21) made of one or more wires coiled about a secondary axis (Z2) normal to the reference plane (R) and forming an implanted receiver (22). As shown in Figures 1 and 7(b), when the electric charger (10) is located adjacent to the AIMD with the primary axis (Z1) positioned coaxially with the secondary axis (Z2) and when the primary coil is fed with electric current, a magnetic link is formed and an electric current is induced in the secondary coil, which serves to power the AIMD or to recharge a rechargeable battery (25) enclosed in the inner volume (Vi).

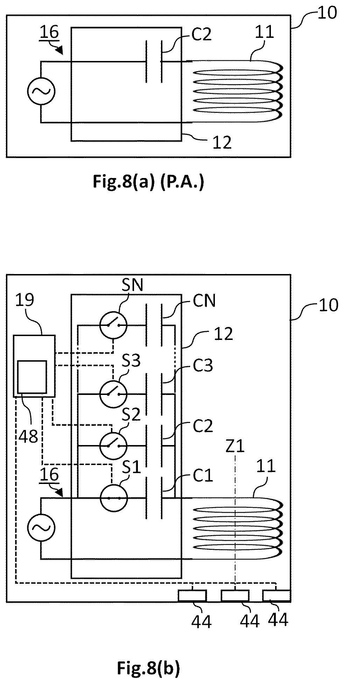

The external charger can comprise a charger housing (10h) enclosing an electrical circuit (16) configured for varying the properties of the electric current fed to the primary coil (11), as shown in Figures 7(b) and 8(b). A processor (19) of the external charger (10) is configured for retrieving the implantation depth (h) of the AIMD (20) measured by the external optical depth measuring device (40) and for controlling the current fed to the primary coil (11) to optimize a power transfer efficiency (P01 P1) between the primary coil (11) of the external charger (10) and the secondary coil (21) of the AIMD as a function of the implantation depth (h).

The power transfer efficiency and the charging current (which affects the charging time) depends *inter alia* on the implantation depth (h). Indeed, the electrical circuit connected to each of the primary and secondary coils (11, 21) contain charging capacitors which capacitance values should be precisely tuned to enhance the resonance between the external charger (10) and the AIMD (20). Resonance occurs when the natural frequency of the system, which depends *inter alia* on the implantation depth (h), is the same as the frequency of the electromagnetic force applied to the system by the oscillating magnetic field (B). Achieving resonance allows maximizing the amplitude of the oscillating response of the system. Figure 8(a) shows a simplified electrical circuit (16) of an external charger of the prior art comprising a single charging capacitor (C2) (the electrical circuit comprises other capacitors performing other functions, but in the present simplified representation of Figure 8(a), only charging capacitor (C2) is shown). Referring to Figure 2, the dashed curve labelled "C2" shows the power transfer efficiency (P0 / P1) as a function of the implantation depth (h) for a system comprising a prior art external charger according to Figure 8(a), with a single charging capacitor (C2) of fixed capacitance. Setting (arbitrarily) a limit of acceptable P0 / P1 value of P0 / P1 = 25%, it can be seen in Figure 2 that the external charger (10) is optimized for a limited C2-implantation depth range (hC2), extending between a lowest boundary depth value (hc2.0) and a highest boundary value (hc2.1). Since external chargers (10) are produced industrially and not tailored to a specific application, such one-fit-them-all external chargers will be suboptimal for all AIMD's implanted at an implantation depth falling out of the C2-implantation depth (hC2). There is no way to change the capacitance of charging capacitor (C2) of the external charger to optimize the power transfer efficiency (P0 / P1). In such conditions, determining the implantation depth (h) of the AIMD may be interesting from an academic point of view or for a number of other reasons, but not for optimizing the power transfer efficiency (P01 P1) between the primary coil (11) of the external charger (10) and the secondary coil (21) of the implanted AIMD (20).

**External charger with several charging capacitors (C1-CN)**

By contrast, the electrical circuit (16) of a preferred external charger (10) of the present invention illustrated in Figure 8(b) comprises at least first and second charging capacitors (C1-CN) of same or different capacitances, which can be individually connected or disconnected to the electrical circuit (16). Typically, the charging capacitors are arranged in parallel with switches (S1-SN) which can be closed or opened to connect or disconnect each corresponding charging capacitor (C1-CN) to the electrical circuit (16). The processor (19) of the external charger (10) is configured for optimizing the power transfer efficiency between the primary coil (11) of the external charger (10) and the secondary coil (21) of the AIMD (20) as a function of the implantation depth (h) by selecting which of charging capacitors (C1, C2) to connect to the electrical circuit (16). Once the best charging capacitors combination is selected, the processor is configured for optimizing the power supplied to the transmitter circuit to yield a nominal charging current of the AIMD.

In a preferred embodiment, the electrical circuit (16) comprises N charging capacitors (C1, C2... CN) wherein N is preferably comprised between 3 and 8, more preferably between 4 and 6. The N charging capacitors can have same or different capacitances. They can be individually connected to or disconnected from the electrical circuit or a selection of charging capacitors forming a group can be connected to or disconnected from the electrical circuit. The processor (19) is configured for determining which combination of one or several of the N charging capacitors (C1, C2... CN) to connect to the electrical circuit (16), such as to optimize the power transfer efficiency as a function of the implantation depth (h), The processor (19) can control the status of the switches (S1-SN) to implement the thus determined combination.

Referring to Figure 2, the solid curve labelled "C1-C4" shows the power transfer efficiency (P0 / P1) as a function of the implantation depth (h) for a system comprising an external charger according to the present invention and to Figure 8(b), with four charging capacitors (C1-C4) of fixed capacitance arranged in parallel with corresponding switches (S1-S4) The combinations of charging capacitors (C1-C4) connected at one time to the electrical circuit (16) was changed for different implantation depths (h) and selected so as to yield the capacitance optimizing the resonance between the primary and secondary coils (11, 21). Setting the limit of acceptable P01 P1 value of P0 / P1 = 25%, as for the dashed curve discussed supra with respect to Figure 8(a) of the prior art, it can be seen in Figure 2 that the external charger (10) is optimized over a much broader C1-4-implantation depth range (hC1-4), extending over both lowest and highest boundary depth values (hc2.0, hc2.1) of the prior art C2-implantation depth range (hC2) discussed supra with respect to the dashed curve of Figure 2 corresponding to the prior art electrical circuit of Figure 8(a),

It can be seen in Figure 2 that the implantation depth range is more than doubled with the external charger (10) having an electrical circuit (16) according to the present invention as illustrated in Figure 8(b), compared with the prior art external charger of Figure 8(a) (i.e., hC1-4 > 2 hC2). This represents a breakthrough as the charging operation of the implanted battery of an AIMD can be performed with a same efficacy regardless of the physiognomy of the patient (lean, fat, muscular, thick / thin featured, etc.) or of the surgeon who implanted the AIMD, which can affect the implantation depth (h) of the AIMD.

**External charger with optical centering system**

In a preferred embodiment, illustrated in Figure 7(b), the external optical depth measuring device (40) is included in the external charger (10), and the intelligence (48) is included in the processor (19). The external charger therefore also comprises the two or more photodetectors (44) or the array of two or more photodetectors of the external optical depth measuring device (40) which are configured for detecting the at least first and second maxima (51m, 52m) of the light projection pattern emitted by the one or more light sources.

In a preferred embodiment, the external charger comprises at least three photodetectors (44) and comprises a module for centring the external charger (10) with respect to the implanted AIMD (20) as described in WO2017202455. The at least three photodetectors (44) form a polygon of N edges, normal to the primary axis (Z1), and which centroid belongs to the primary axis (Z1). The module comprises an indicator indicating how the external charger is to be displaced over the surface of the skin of a patient to position the external charger with the primary axis, (Z1), being coaxial with the secondary axis (Z2), of the AIMD, as a function of the energy received by each of the at least three photodetectors from the light beam emitted by a central light source of the AIMD.

The position of the secondary axis (Z2) centred on the secondary coil (21) of the AIMD can be univocally determined from the position of a central light source. Preferably, the central light source belongs to the secondary axis (Z2) or is offset therefrom by a known distance and angle. The central light source emits a light beam perpendicular to the reference plane (R), i.e., the axis angle of the central light beam must be 0°. The central light source can be different from the one or more light sources (51, 52) used for determining the implantation depth (h). It is, however, preferred that the central light source be one of the one or more light sources (51, 52) which must have an axis angle, $\alpha 1 = 0°$. For example, the central light source could be the first light source (51) of Figure 5.

With this embodiment, the external charger (10) including the external optical depth measuring device (40) can be used for continuously and sequentially

- aligning the external charger with the AIMD (20),

- determining the implantation depth (h) of the AIMD,

- optimizing the electrical circuit (16) to approach and preferably reach resonance and

- finally, recharging the implanted battery as efficiently and comfortably as possible to date. A rapid battery charging operation is beneficial to the comfort of the patients.

**METHOD FOR DETERMINING AN IMPLANTATION DEPTH (h) OF AN AIMD**

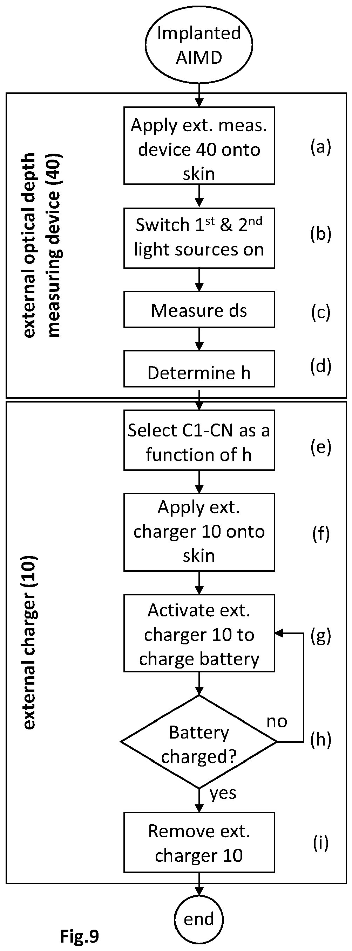

The present invention also concerns a method for determining an implantation depth (h) separating an external optical measuring device (40) as described supra from an AIMD implanted in a patient. The method is illustrated in the flowchart of Figure 9 and requires that the implanted AIMD be as discussed supra, comprising one or more light sources (51, 52) as defined supra. As shown in Figure 9(a), the external optical depth measuring device (40) is applied onto the skin of the patient, vis-à-vis the AIMD. The controller activates the one or more light sources (51, 52) of the AIMD (cf. Figure 9(b)) and measures the maxima distance (ds) separating the at least first and second maxima (51m, 52m) (cf. Figure 9(c)). As shown in Figure 9(d), the value of the implantation depth can then be determined as explained with reference to Figures 3 to 5.

Once the implantation depth (h) has been determined, the power transfer efficiency between a primary coil (11) of an external charger (10) and a secondary coil (55) of the AIMD (20) can be optimized as a function of the thus determined implantation depth (h), as follows. An external charger (10) as defined above is provided. Based on the implantation depth (h) thus determined, an optimal electric current is determined to be fed to the primary coil (11) as a function of the implantation depth (h) (cf. Figure 9(e)), to optimize the power transfer efficiency (P0 / P1) between the primary coil (11) of the external charger (10) and the secondary coil (21) of the AIMD. As indicated in Figure 9(f), the external charger (10) can be laid onto the skin vis-à-vis the AIMD and the optimal electric current can be fed to the primary coil (11) to induce a current in the secondary coil (21) (cf. Figure 9(g)). The feeding of electric current is carried out until a desired level of charging of the battery is reached (cf. loop (g)-(h)), at which point the external charger (10) can be removed, as indicated in Figure 9(i)).

The optimal current fed to the primary coil (11) can be controlled by selecting one or more charging capacitors (C1-CN) to connect to the electrical circuit (16), such as to optimize the power transfer efficiency between the primary coil (11) of the external charger (10) and the secondary coil (21) of the AIMD (20) as a function of the distance thus determined. The controller controls the switches (S1-SN) to connect or disconnect the corresponding charging capacitors to the electrical circuit.

| REF. # | DESCRIPTION |
|---|---|
| 3s | Skin |
| 10 | External charger |
| 11 | Primary coil |
| 12 | Transmitter |
| 16 | Electrical circuit of the external charger |
| 19 | Processor (external charger) |
| 20 | AIMD |
| 20e | Encapsulation unit |
| 20h | Housing of AIMD |
| 20m | Main surface of housing |
| 21 | Secondary coil |
| 22 | Receiver |
| 25 | Implanted rechargeable battery |
| 40 | External optical depth measuring device |
| 40h | Housing of external charger |
| 44 | Photodetector of the external optical depth measuring device |
| 48 | Intelligence of the external optical depth measuring device |
| 51, 52 | First and second light sources |
| 51m, 52m | First and second maxima |
| 51s, 52s | First and second light projection patterns |
|  |  |
| C1-CN | First to $N^{th}$ charging capacitors |
| d0 | Distance separating first and second light sources |
| D1, D2 | Distance separating the first and second light sources from central normal axis n0 |
| ds | Maxima distance |
| h | Implantation depth and distance between primary and secondary coils |
| hC1-4 | C1-4-implantation depth range |
| hC2 | C2-implantation depth range |
| hc2.0 | hC2 lower boundary |
| hc2.1 | hC2 upper boundary |
| n0 | Central normal axis |
| n1, n2 | Normal axes intercepting first and second light sources, respectively |

(continued)

| REF. # | DESCRIPTION |
|---|---|
| S1-SN | First to $N^{th}$ switches |
| Vi | Inner volume |
| X1, X2 | First and second emission axes |
| X, Y, Z | Space reference axes |
| Z1-Z2 | Primary and secondary axes of the corresponding coils |
| $\alpha 1, \alpha 2$ | Axis angles |

**Claims**

1. System for optically measuring an implantation depth (d) of an active implantable medical device (AIMD) below a skin (3s) of a patient implanted with the AIMD, wherein the AIMD (20) comprises,

   • an encapsulation unit (20e) suitable for being subcutaneously implanted with a reference plane (R) of the encapsulation unit being substantially parallel to the skin (3s) of the patient when implanted in the patient and comprising a housing (20h) comprising walls defining an inner volume (Vi) sealed from an outer environment by the walls and defining a first main surface (20m) configured for facing the skin of the patient when implanted in the patient,
   **characterized in that**, the AIMD comprises,

     • one or more light sources (51, 52) configured for emitting one or more light beams from the main surface (20m) towards the outer environment to form a light projection pattern (51s, 52s) at a level of the skin (3s) of the patient, **characterized by** an intensity profile comprising at least first and second maxima (51m, 52m) separated from one another by a maxima distance (ds), wherein
     • the one or more light sources are configured for emitting one or more light beams along at least two emission axes (X1 , X2) defined as the axes extending from one or more emission points (51, 52) of the one or more light sources to the corresponding at least first and second maxima (51m, 52m) of the light projection pattern (51s, 52s) at the level of the skin (3s) of the patient, wherein the at least two emission axes (X1 , X2) are non-parallel to one another, and form at least first and second axis angles ($\alpha 1, \alpha 2$) relative to a normal axis (nl ; n2) perpendicular to the reference plane (R), each comprised between 0° and 88°, preferably between 10° and 60°, more preferably between 20° and 45°, and

   in that, the system comprises an external optical depth measuring device (40) comprising,

     • a controller configured for activating the one or more light sources of the AIMD, and
     • two or more photodetectors (44) or an array of two or more photodetectors configured for detecting the at least first and second maxima (51m, 52m) of the light projection pattern emitted by the one or more light sources, and
     • an intelligence (48) configured for,

       o determining the respective positions of the first and second maxima (51m, 52m),
       o measuring the maxima distance (ds) between the first and second maxima (51m, 52m) at the level of the skin (3s) of the patient, and
       o determining from the maxima distance (ds) the implantation depth (h) of the AIMD below the skin (3s) of the patient.

2. System according to claim 1, wherein the implantation depth (h) is determined with any one of the following expressions (1) or (1a),

$$h = \frac{ds}{\tan \alpha 1 + \tan \alpha 2} - h0, \text{ or} \qquad (1)$$

$$h = \frac{ds - d0}{\tan \alpha 1 + \tan \alpha 2} \qquad (1a)$$

wherein

- h0 is a distance measured along a central normal axis (n0) perpendicular to the reference plane (R) between the intersection point of the first and second emission axes (XI, X2) and the main surface (20m), and
- d0 is a distance separating the first from the second light sources (51, 52).

3. System according to claim 2, wherein the first and second axis angles ($\alpha 1, \alpha 2$) are equal and the implantation depth (h) is determined with any one of the following expressions (3) or (3a),

$$h = \frac{ds}{2 \tan \alpha 1} - h0 \qquad (3)$$

$$h = \frac{ds - d0}{2 \tan \alpha 1} \qquad (3a)$$

4. System according to claim 2, wherein the first axis angle ($\alpha 1$) is equal to zero and is different from the second axis angle ($a2$) and wherein the implantation depth (h) is determined with any one of the following expressions (4) or (4a),

$$h = \frac{ds}{\tan \alpha 2} - h0 \qquad (4)$$

$$h = \frac{ds - d0}{\tan \alpha 2} \qquad (4)$$

5. System according to any one of the preceding claims, comprising either,

- at least first and second light sources (51, 52) separated from one another by a distance (d0) and each configured for emitting a light beam along the corresponding at least first and second emission axes (X1 , X2), or
- a single light source (51) configured for emitting a light beam **characterized by** the at least first and second maxima (51m, 52m).

6. System according to any one of the preceding claims,

- comprising an external charger (10) comprising or connectable to a source of power for feeding alternative current to a primary coil (1 1) made of one or more wires coiled about a primary axis (Z1) and forming an external emitter (12) of magnetic waves,
- wherein the AIMD (20) comprises a secondary coil (21) made of one or more wires coiled about a secondary axis (Z2) normal to the reference plane (R) and forming an internal receiver (22),

such that when the electric charger is located adjacent to the AIMD with the primary axis (Z1) positioned coaxially to the secondary axis (Z2) and when the primary coil is fed with alternative current, a magnetic link is formed and an alternative current induced in the secondary coil is optimized, which serves to power the AIMD or to recharge a rechargeable battery (25) enclosed in the inner volume (Vi).

7. System according to the preceding claim 6, wherein the external charger comprises,

- an electrical circuit (16) configured for varying the properties of the alternative current fed to the primary coil (11),
- a processor (1 9) configured for retrieving the implantation depth (h) of the AIMD (20) measured by the external optical depth measuring device (40) and for controlling the alternative current fed to the primary coil (1 1) to optimize a power transfer efficiency between the primary coil (11) of the external charger (10) and the secondary coil (21) of the AIMD as a function of the implantation depth (h).

8. System according to the preceding claim 7 wherein,

- the electrical circuit (16) comprises at least first and second charging capacitors (C1, C2) of given capacitances, which can be individually connected or disconnected to the electrical circuit,
• the processor (1 9) is configured for optimizing the power transfer efficiency between the primary coil (11) of the external charger (10) and the secondary coil (21) of the AIMD (20) as a function of the implantation depth (h) by selecting which of the first and / or second capacitance (C1, C2) to connect to the electrical circuit (16).

9. System according to the preceding claim 8, wherein the electrical circuit (16) comprises N charging capacitors (C1, C2... CN) of same or different charging capacitors, which can be connected or disconnected individually or in groups of charging capacitors to the electrical circuit, and wherein the processor (19) is configured for determining which combination of one or several of the N charging capacitors (Cl, C2... CN) to connect to the electrical circuit (16), such as to optimize the power transfer efficiency as a function of the implantation depth (h), wherein N is preferably comprised between 3 and 8, more preferably between 4 and 6.

10. System according to any one of claim 6 to 9, wherein the external optical depth measuring device (40) is included in the external charger (10), and wherein the intelligence (48) is included in the processor (19).

11. Method for determining an implantation depth (h) separating an external optical measuring device (40) from an active implantable medical device (AIMD) implanted in a patient, comprising the following steps,

• providing an already implanted AIMD (20) as defined in a system according to any one of claims 1 to 10, whose main surface (20m) is separated from an outer environment by a distance to be determined, by a membrane substantially parallel to the reference plane (R),
• applying an external depth measuring device (40) as defined in the system according to any one of claims 1 to 9 onto the membrane, vis-à-vis the AIMD,
• activating with the controller the one or more light sources (51, 52) of the AIMD,
• measuring the maxima distance (ds) and determining therefrom the value of the distance.

12. Method according to the preceding claim, further comprising the following steps for optimizing a power transfer efficiency between a primary coil (1 1) of an external charger (10) and a secondary coil (55) of the AIMD (20) as a function of the implantation depth (h),

• providing an external charger (10) as defined in the system according to claim 10,
• retrieving the thus determined distance, and
• determining an optimal alternative current to be fed to the primary coil (11) as a function of the distance, to optimize the power transfer efficiency (P0 / P1) between the primary coil (11) of the external charger (10) and the secondary coil (21) of the AIMD,
• applying the external charger (10) onto the membrane vis-à-vis the AIMD and feeding the optimal alternative current to the primary coil (1 1).

13. Method according to the preceding claim, wherein the system is according to claim 8 or 9, and wherein controlling the alternative current fed to the primary coil (11) comprises selecting one or more charging capacitors (C1-CN) to connect to the electrical circuit (16), such as to optimize the power transfer efficiency between the primary coil (11) of the external charger (10) and the secondary coil (21) of the AIMD (20) as a function of the distance thus determined.

**Patentansprüche**

1. System zum optischen Messen einer Implantationstiefe (d) eines aktiven implantierbaren medizinischen Geräts (AIMD) unter einer Haut (3s) eines Patienten, in den das AIMD implantiert ist, wobei das AIMD (20) Folgendes umfasst:

• eine Verkapselungseinheit (20e), die dafür geeignet ist, um subkutan implantiert zu werden, wobei eine Bezugsebene (R) der Verkapselungseinheit im Wesentlichen parallel zu der Haut (3s) des Patienten ist, wenn sie in dem Patienten implantiert ist, und ein Gehäuse (20h) umfasst, das Wände umfasst, die einen von einer äußeren Umgebung durch die Wände abgedichteten Innenraum (Vi) definieren und eine erste Hauptoberfläche (20m) definieren, die so ausgebildet ist, dass sie der Haut des Patienten zugewandt ist, wenn sie in dem Patienten implantiert ist,
**dadurch gekennzeichnet, dass** das AIMD Folgendes umfasst:

• eine oder mehrere Lichtquellen (51, 52), die zum Aussenden eines oder mehrerer Lichtstrahlen von der Hauptoberfläche (20m) zu der äußeren Umgebung hin ausgebildet ist/sind, um ein Lichtprojektionsmuster (51s, 52s) auf einer Ebene der Haut (3s) des Patienten zu bilden, **gekennzeichnet durch** ein Intensitätsprofil, das mindestens ein erstes und ein zweites Maximum (51m, 52m), die um einen Maxima-Abstand (ds) voneinander getrennt sind, umfasst, wobei
• die eine oder die mehreren Lichtquellen zum Aussenden eines oder mehrerer Lichtstrahlen entlang mindestens zwei Aussendeachsen (X1, X2) ausgebildet ist/sind, die als die Achsen definiert sind, die sich von einem oder mehreren Aussendepunkten (51, 52) der einen oder der mehreren Lichtquellen zu dem entsprechenden mindestens einen ersten und dem entsprechenden mindestens einen zweiten Maximum (51m, 52m) des Lichtprojektionsmusters (51s, 52s) auf der Ebene der Haut (3s) des Patienten erstrecken, wobei die mindestens zwei Aussendeachsen (X1, X2) zueinander nicht parallel sind und mindestens einen ersten und einen zweiten Achsenwinkel ($\alpha$1, $\alpha$2) relativ zu einer senkrecht auf der Bezugsebene (R) stehenden Normalachse (n1; n2) bilden, die je zwischen 0° und 88°, vorzugsweise zwischen 10° und 60°, besonders vorzugsweise zwischen 20° und 45° umfasst sind, und

**dadurch, dass** das System eine externe Vorrichtung (40) zum Messen einer optischen Tiefe umfasst, umfassend

• eine Steuerung, die zum Einschalten der einen oder der mehreren Lichtquellen des AIMD ausgebildet ist, und
• zwei oder mehr Photodetektoren (44) oder ein Array von zwei oder mehr Photodetektoren, die zum Detektieren des mindestens einen ersten und des mindestens einen zweiten Maximums (51m, 52m) des von der einen oder den mehreren Lichtquellen ausgesendeten Lichtprojektionsmusters ausgebildet sind, und
• eine Intelligenz (48), die für Folgendes ausgebildet ist:

◦ Bestimmen der jeweiligen Positionen des ersten und des zweiten Maximums (51m, 52m),
◦ Messen des Maxima-Abstands (ds) zwischen dem ersten und dem zweiten Maximum (51m, 52m) auf der Ebene der Haut (3s) des Patienten und
◦ Bestimmen, aus dem Maxima-Abstand (ds), der Implantationstiefe (h) des AIMD unter der Haut (3s) des Patienten.

2. System nach Anspruch 1, wobei die Implantationstiefe (h) mit einem beliebigen der folgenden Ausdrücke (1) oder (1a)

$$h = \frac{ds}{\tan \alpha 1 + \tan \alpha 2} - h0 \quad \texttt{oder} \qquad (1)$$

$$h = \frac{ds - d0}{\tan \alpha 1 + \tan \alpha 2} \qquad (\texttt{1a})$$

bestimmt wird, wobei

• h0 ein Abstand ist, der entlang einer mittleren Normalachse (n0), die senkrecht auf der Bezugsebene (R) zwischen dem Schnittpunkt der ersten und der zweiten Aussendeachse (X1, X2) und der Hauptoberfläche (20m) steht, gemessen wird, und
• d0 ein Abstand ist, um den die erste von der zweiten Lichtquelle (51, 52) getrennt ist.

3. System nach Anspruch 2, wobei der erste und der zweite Achsenwinkel ($\alpha$1, $\alpha$2) gleich sind und die Implantationstiefe (h) mit einem beliebigen der folgenden Ausdrücke (3) oder (3a)

$$h = \frac{ds}{2 \tan \alpha 1} - h0 \qquad (3)$$

$$h = \frac{ds - d0}{2 \tan \alpha 1} \qquad (\texttt{3a})$$

bestimmt wird.

4. System nach Anspruch 2, wobei der erste Achsenwinkel ($\alpha$1) gleich null ist und sich von dem zweiten Achsenwinkel ($\alpha$2) unterscheidet und wobei die Implantationstiefe (h) mit einem beliebigen der folgenden Ausdrücke (4) oder (4a)

$$h = \frac{ds}{\tan \alpha2} - h0 \qquad (4)$$

$$h = \frac{ds - d0}{\tan \alpha2} \qquad (4)$$

bestimmt wird.

5. System nach einem der vorhergehenden Ansprüche, umfassend entweder

• mindestens eine erste und eine zweite Lichtquelle (51, 52), die um einen Abstand (d0) voneinander getrennt und je zum Aussenden eines Lichtstrahls entlang der entsprechenden mindestens einen ersten und der entsprechenden mindestens einen zweiten Aussendeachse (X1, X2) ausgebildet sind, oder
• eine einzige Lichtquelle (51), die zum Aussenden eines Lichtstrahls ausgebildet ist, der durch das mindestens eine erste und das mindestens eine zweite Maximum (51m, 52m) gekennzeichnet ist.

6. System nach einem der vorhergehenden Ansprüche,

• das ein externes Ladegerät (10) umfasst, das eine Leistungsquelle zum Zuführen von alternativem Strom zu einer Primärspule (11), die aus einem oder mehreren um eine Primärachse (Z1) gewickelten Drähten hergestellt ist und einen externen Sender (12) elektromagnetischer Wellen bildet, umfasst oder an diese Leistungsquelle anschließbar ist,
• wobei das AIMD (20) eine Sekundärspule (21) umfasst, die aus einem oder mehreren Drähten, die um eine zu der Bezugsebene (R) normale Sekundärachse (Z2) gewickelt ist/sind, besteht und einen internen Empfänger (22) bildet,

sodass, wenn sich das Ladegerät neben dem AIMD befindet und die Primärachse (Z1) koaxial zu der Sekundärachse (Z2) positioniert ist und wenn der Primärspule alternativer Strom zugeführt wird, eine magnetische Verbindung gebildet wird und ein in der Sekundärspule induzierter alternativer Strom optimiert wird, der dazu dient, das AIMD mit Strom zu versorgen oder eine in dem Innenraum (Vi) eingeschlossene wiederaufladbare Batterie (25) wiederaufzuladen.

7. System nach dem vorhergehenden Anspruch 6, wobei das externe Ladegerät Folgendes umfasst:

• einen Stromkreis (16), der zum Variieren der Eigenschaften des der Primärspule (11) zugeführten alternativen Stroms ausgebildet ist,
• einen Prozessor (19), der zum Abrufen der Implantationstiefe (h) des AIMD (20), die von der externen Vorrichtung (40) zum Messen einer optischen Tiefe gemessen wird, und zum Steuern des der Primärspule (11) zugeführten alternativen Stroms ausgebildet ist, um einen Leistungsübertragungswirkungsgrad zwischen der Primärspule (11) des externen Ladegeräts (10) und der Sekundärspule (21) des AIMD in Abhängigkeit von der Implantationstiefe (h) zu optimieren.

8. System nach dem vorhergehenden Anspruch 7, wobei

• der Stromkreis (16) mindestens einen ersten und einen zweiten Ladekondensator (C1, C2) mit vorgegebenen Kapazitäten umfasst, die einzeln an den Stromkreis angeschlossen oder von ihm getrennt werden können,
• der Prozessor (19) zum Optimieren des Leistungsübertragungswirkungsgrads zwischen der Primärspule (11) des externen Ladegeräts (10) und der Sekundärspule (21) des AIMD (20) in Abhängigkeit von der Implantationstiefe (h) durch Auswählen, welcher von dem ersten und/oder dem zweiten Kondensator (C1, C2) an den Stromkreis (16) anzuschließen ist, ausgebildet ist.

9. System nach dem vorhergehenden Anspruch 8, wobei der Stromkreis (16) N Ladekondensatoren (C1, C2 ... CN) von gleichen oder unterschiedlichen Ladekondensatoren umfasst, die einzeln oder in Gruppen von Ladekondensatoren an den Stromkreis angeschlossen oder von ihm getrennt werden können, und wobei der Prozessor (19) ausgebildet ist, um zu bestimmen, welche Kombination eines oder mehrerer der N Ladekondensatoren (C1, C2 ... CN) an den Stromkreis (16) anzuschließen ist, etwa um den Leistungsübertragungswirkungsgrad in Abhängigkeit von der Implantationstiefe (h) zu optimieren, wobei N vorzugsweise zwischen 3 und 8, besonders vorzugsweise zwischen 4 und 6 umfasst ist.

10. System nach einem der Ansprüche 6 bis 9, wobei die externe Vorrichtung (40) zum Messen einer optischen Tiefe in dem externen Ladegerät (10) enthalten ist und wobei die Intelligenz (48) in dem Prozessor (19) enthalten ist.

11. Verfahren zum Bestimmen einer Implantationstiefe (h), bei dem eine externe Vorrichtung (40) zum Messen einer optischen Tiefe von einem aktiven implantierbaren medizinischen Gerät (AIMD), das in einem Patienten implantiert ist, getrennt ist und das die folgenden Schritte umfasst:

   • Bereitstellen eines bereits implantierten AIMD (20), wie in einem System nach einem der Ansprüche 1 bis 10 definiert, dessen Hauptoberfläche (20m) durch eine im Wesentlichen zu der Bezugsebene (R) parallele Membran um einen zu bestimmenden Abstand von einer äußeren Umgebung getrennt ist,
   • Anlegen einer externen Vorrichtung (40) zum Messen einer optischen Tiefe, wie in dem System nach einem der Ansprüche 1 bis 9 definiert, an die Membran, gegenüber dem AIMD,
   • Einschalten, mit der Steuerung, der einen oder der mehreren Lichtquellen (51, 52) des AIMD,
   • Messen des Maxima-Abstands (ds) und Bestimmen des Werts des Abstands daraus.

12. Verfahren nach dem vorhergehenden Anspruch, das ferner die folgenden Schritte zum Optimieren eines Leistungsübertragungswirkungsgrads zwischen einer Primärspule (11) eines externen Ladegeräts (10) und einer Sekundärspule (55) des AIMD (20) in Abhängigkeit von der Implantationstiefe (h) umfasst:

   • Bereitstellen eines externen Ladegeräts (10), wie in dem System nach Anspruch 10 definiert,
   • Abrufen des hierdurch bestimmten Abstands und
   • Bestimmen eines optimalen alternativen Stroms zum Zuführen zu der Primärspule (11) in Abhängigkeit von dem Abstand, um den Leistungsübertragungswirkungsgrad (P0 / P1) zwischen der Primärspule (11) des externen Ladegeräts (10) und der Sekundärspule (21) des AIMD zu optimieren,
   • Anlegen des externen Ladegeräts (10) an die Membran gegenüber dem AIMD und Zuführen des optimalen alternativen Stroms zu der Primärspule (11).

13. Verfahren nach dem vorhergehenden Anspruch, wobei das System ein System nach Anspruch 8 oder 9 ist und wobei das Steuern des der Primärspule (11) zugeführten alternativen Stroms umfasst, dass ein oder mehrere Ladekondensatoren (C1-CN) zum Anschließen an den Stromkreis (16) ausgewählt wird/werden, etwa um den Leistungsübertragungswirkungsgrad zwischen der Primärspule (11) des externen Ladegeräts (10) und der Sekundärspule (21) des AIMD (20) in Abhängigkeit von dem hierdurch bestimmten Abstand zu optimieren.

**Revendications**

1. Système de mesure optique d'une profondeur d'implantation (d) d'un dispositif médical implantable actif (AIMD) sous la peau (3s) d'un patient chez qui l'AIMD a été implanté, l'AIMD (20) comprenant :

   une unité d'encapsulation (20e) apte à être implantée par voie sous-cutanée avec un plan de référence (R) de l'unité d'encapsulation sensiblement parallèle à la peau (3s) du patient lorsqu'elle est implantée chez le patient, et comprenant un boîtier (20h) comprenant des parois définissant un volume intérieur (Vi) isolé d'un environnement extérieur par les parois et définissant une première surface principale (20m) configurée pour faire face à la peau du patient lorsqu'elle est implantée chez le patient, le système étant **caractérisé en ce que** l'AIMD comprend :

   une ou plusieurs sources de lumière (51, 52) configurées pour émettre un ou plusieurs faisceaux lumineux depuis la surface principale (20m) vers l'environnement extérieur afin de former un motif de projection de lumière (51s, 52s) à un niveau de la peau (3s) du patient, **caractérisé par** un profil d'intensité comprenant au moins des première et seconde valeurs maximales (51m, 52m) séparées l'une de l'autre par une distance maximale (ds),

la ou les sources de lumière étant configurées pour émettre un ou plusieurs faisceaux lumineux le long d'au moins deux axes d'émission (X1, X2) définis comme les axes s'étendant d'un ou plusieurs points d'émission (51, 52) de la ou des sources de lumière aux au moins première et seconde valeurs maximales (51m, 52m) correspondantes du motif de projection de lumière (51s, 52s) au niveau de la peau (3s) du patient, les au moins deux axes d'émission (X1, X2) n'étant pas parallèles entre eux et formant au moins des premier et second angles d'axe ($\alpha$1, $\alpha$2) par rapport à un axe normal (n1, n2) perpendiculaire au plan de référence (R), chacun étant compris entre 0° et 88°, de préférence entre 10° et 60°, plus préférablement entre 20° et 45°, et

en ce que le système comprend un dispositif optique externe de mesure de profondeur (40) comprenant :

un dispositif de commande configuré pour activer la ou les sources de lumière de l'AIMD, et
deux photodétecteurs (44) ou plus ou un réseau de deux photodétecteurs ou plus configurés pour détecter les au moins première et seconde valeurs maximales (51m, 52m) du motif de projection de lumière émis par la ou les sources de lumière, et
une intelligence (48) configurée pour :

déterminer les positions respectives des première et seconde valeurs maximales (51m, 52m),
mesurer la distance maximale (ds) entre les première et seconde valeurs maximales (51m, 52m) au niveau de la peau (3s) du patient, et
déterminer, à partir de la distance maximale (ds), la profondeur d'implantation (h) de l'AIMD sous la peau (3s) du patient.

2. Système selon la revendication 1, dans lequel la profondeur d'implantation (h) est déterminée par l'une quelconque des expressions suivantes (1) ou (1à) :

$$h = \frac{ds}{\tan \alpha 1 + \tan \alpha 2} - h0 \qquad (1),$$

ou

$$h = \frac{ds - d0}{\tan \alpha 1 + \tan \alpha 2} \qquad (1a)$$

où :

h0 est une distance mesurée le long d'un axe normal central (n0) perpendiculaire au plan de référence (R) entre le point d'intersection des premier et second axes d'émission (X1, X2) et de la surface principale (20m), et
d0 est une distance séparant la première de la seconde source de lumière (51, 52).

3. Système selon la revendication 2, dans lequel les premier et second angles d'axe ($\alpha$1, $\alpha$2) sont égaux et la profondeur d'implantation (h) est déterminée par l'une quelconque des expressions suivantes (3) ou (3a) :

$$h = \frac{ds}{2\tan \alpha 1} - h0 \qquad (3)$$

$$h = \frac{ds - d0}{2\tan \alpha 1} \qquad (3a)$$

4. Système selon la revendication 2, dans lequel le premier angle d'axe ($\alpha$1) est égal à zéro et est différent du second angle d'axe ($\alpha$2), et dans lequel la profondeur d'implantation (h) est déterminée par l'une quelconque des expressions suivantes (4) ou (4a) :

$$h = \frac{ds}{\tan \alpha 2} - h0 \qquad (4)$$

$$h = \frac{ds - d0}{\tan \alpha 2} \qquad (4)$$

5. Système selon l'une quelconque des revendications précédentes, comprenant soit :

> au moins des première et seconde sources de lumière (51, 52) séparées l'une de l'autre par une distance (d0) et configurées chacune pour émettre un faisceau lumineux le long des au moins premier et second axes d'émission (X1, X2) correspondants, soit
> une seule source de lumière (51) configurée pour émettre un faisceau lumineux **caractérisé par** les au moins première seconde valeurs maximales (51m, 52m).

6. Système selon l'une quelconque des revendications précédentes,

> comprenant un chargeur externe (10) comprenant une source d'énergie ou pouvant être connecté à celle-ci pour alimenter en courant alternatif une bobine primaire (11) constituée d'un ou plusieurs fils enroulés autour d'un axe primaire (Z1) et formant un émetteur externe (12) d'ondes magnétiques,
> l'AIMD (20) comprenant une bobine secondaire (21) constituée d'un ou plusieurs fils enroulés autour d'un axe secondaire (Z2) normal au plan de référence (R) et formant un récepteur interne (22),
> de telle sorte que lorsque le chargeur électrique est situé à proximité de l'AIMD avec l'axe primaire (Z1) positionné coaxialement à l'axe secondaire (Z2) et lorsque la bobine primaire est alimentée en courant alternatif, une liaison magnétique soit formée et un courant alternatif induit dans la bobine secondaire soit optimisé, ce qui permet d'alimenter l'AIMD ou de recharger une batterie rechargeable (25) enfermée dans le volume intérieur (Vi).

7. Système selon la revendication 6 précédente, dans lequel le chargeur externe comprend :

> un circuit électrique (16) configuré pour faire varier les propriétés du courant alternatif alimentant la bobine primaire (11),
> un processeur (19) configuré pour récupérer la profondeur d'implantation (h) de l'AIMD (20) mesurée par le dispositif optique externe de mesure de profondeur (40) et pour commander le courant alternatif alimentant la bobine primaire (11) afin d'optimiser un rendement de transfert d'énergie entre la bobine primaire (11) du chargeur externe (10) et la bobine secondaire (21) de l'AIMD en fonction de la profondeur d'implantation (h).

8. Système selon la revendication 7 précédente, dans lequel :

> le circuit électrique (16) comprend au moins des premier et second condensateurs de recharge (C1, C2) de capacités données, qui peuvent être connectés individuellement au circuit électrique ou déconnectées individuellement de celui-ci,
> le processeur (19) étant configuré pour optimiser le rendement de transfert d'énergie entre la bobine primaire (11) du chargeur externe (10) et la bobine secondaire (21) de l'AIMD (20) en fonction de la profondeur d'implantation (h) en sélectionnant lequel des premier et/ou second condensateurs (C1, C2) connecter au circuit électrique (16).

9. Système selon la revendication 8 précédente, dans lequel le circuit électrique (16) comprend N condensateurs de recharge (C1, C2, ..., CN) de capacités de recharge identiques ou différentes, qui peuvent être connectés individuellement ou en groupes de condensateurs de recharge au circuit électrique ou bien déconnectés individuellement ou en groupes de condensateurs de recharge de celui-ci, et dans lequel le processeur (19) est configuré pour déterminer quelle combinaison d'un ou plusieurs des N condensateurs de recharge (C1, C2, ..., CN) connecter au circuit électrique (16), de manière à optimiser le rendement de transfert d'énergie en fonction de la profondeur d'implantation (h), N étant de préférence compris entre 3 et 8, plus préférablement entre 4 et 6.

10. Système selon l'une quelconque des revendications 6 à 9, dans lequel le dispositif optique externe de mesure de profondeur (40) est compris dans le chargeur externe (10), et dans lequel l'intelligence (48) est comprise dans le processeur (19).

**11.** Procédé de détermination d'une profondeur d'implantation (h) séparant un dispositif optique externe de mesure (40) d'un dispositif médical implantable actif (AIMD) implanté chez un patient, le procédé comprenant les étapes suivantes consistant à :

fournir un AIMD (20) déjà implanté, tel que défini dans un système selon l'une quelconque des revendications 1 à 10, dont la surface principale (20m) est séparée d'un environnement extérieur par une distance à déterminer, par une membrane sensiblement parallèle au plan de référence (R),
appliquer sur la membrane un dispositif externe de mesure de profondeur (40) tel que défini dans le système selon l'une quelconque des revendications 1 à 9, en regard de l'AIMD,
activer à l'aide du dispositif de commande la ou les sources de lumière (51, 52) de l'AIMD,
mesurer la distance maximale (ds) et déterminer à partir de celle-ci la valeur de la distance.

**12.** Procédé selon la revendication précédente, comprenant en outre les étapes suivantes pour optimiser un rendement de transfert d'énergie entre une bobine primaire (11) d'un chargeur externe (10) et une bobine secondaire (55) de l'AIMD (20) en fonction de la profondeur d'implantation (h) :

fournir un chargeur externe (10) tel que défini dans le système selon la revendication 10,
récupérer la distance ainsi déterminée, et
déterminer un courant alternatif optimal pour alimenter la bobine primaire (11) en fonction de la distance, afin d'optimiser le rendement de transfert d'énergie (P0/P1) entre la bobine primaire (11) du chargeur externe (10) et la bobine secondaire (21) de l'AIMD,
appliquer le chargeur externe (10) sur la membrane en regard de l'AIMD et alimenter la bobine primaire (11) avec le courant alternatif optimal.

**13.** Procédé selon la revendication précédente, dans lequel le système est selon la revendication 8 ou 9, et dans lequel la commande du courant alternatif alimentant la bobine primaire (11) comprend l'étape consistant à sélectionner un ou plusieurs condensateurs de recharge (C1-CN) à connecter au circuit électrique (16), de manière à optimiser le rendement de transfert d'énergie entre la bobine primaire (11) du chargeur externe (10) et la bobine secondaire (21) de l'AIMD (20) en fonction de la distance ainsi déterminée.

FIG.1

FIG.2

$\forall \; \alpha 1, \alpha 2$

$$h = \frac{ds}{\tan \alpha 1 + \tan \alpha 2} - h0$$

$$h = \frac{ds - d0}{\tan \alpha 1 + \tan \alpha 2}$$

**FIG.3(a)**

$\alpha 1 = 30°$
$\alpha 2 = 20°$
$d0 = 6 \text{ mm}$

**FIG.3(b)**

FIG.3(c)

$\alpha 1 = \alpha 2$

$$h = \frac{ds}{2 \tan \alpha 1} - h0$$

$$h = \frac{ds - d0}{2 \tan \alpha 1}$$

FIG.4

$$\alpha 1 = 0°$$

$$h = \frac{ds}{\tan \alpha 2} - h0$$

$$h = \frac{ds - d0}{\tan \alpha 2}$$

FIG.5

**FIG.6**

FIG.7(a)

FIG.7(b)

**Fig.8(a) (P.A.)**

**Fig.8(b)**

Implanted
AIMD

**external optical depth measuring device (40)**

Apply ext. meas. device 40 onto skin | (a)

Switch 1st & 2nd light sources on | (b)

Measure ds | (c)

Determine h | (d)

**external charger (10)**

Select C1-CN as a function of h | (e)

Apply ext. charger 10 onto skin | (f)

Activate ext. charger 10 to charge battery | (g)

Battery charged? | (h)

no

yes

Remove ext. charger 10 | (i)

end

**Fig.9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017202455 A
- US 20080262468 A
- US 20140203823 A
- WO 2016131492 A